(19) Europäisches Patentamt
European Patent Office
Office européen des brevets

(11) **EP 3 059 645 B1**

(12) **EUROPEAN PATENT SPECIFICATION**

(45) Date of publication and mention
of the grant of the patent:
**01.06.2022 Bulletin 2022/22**

(21) Application number: **14854626.0**

(22) Date of filing: **14.10.2014**

(51) International Patent Classification (IPC):
**G16C 99/00** (2019.01)    **G16C 20/10** (2019.01)
**C12P 5/02** (2006.01)    **C02F 11/04** (2006.01)
**C02F 3/28** (2006.01)    **G05B 13/04** (2006.01)

(52) Cooperative Patent Classification (CPC):
**C02F 3/28; C12M 21/04; C12M 41/48; C12P 5/023;**
**G16C 20/10; G16C 99/00;** A01C 3/023;
C02F 3/2846; C02F 11/04; C02F 2103/20;
C02F 2209/006; C02F 2209/05; C02F 2209/07;
C02F 2209/16; Y02E 50/30

(86) International application number:
**PCT/ES2014/070777**

(87) International publication number:
**WO 2015/055880 (23.04.2015 Gazette 2015/16)**

(54) **METHOD AND COMPUTER PROGRAM PRODUCT FOR CONTROLLING ANAEROBIC CODIGESTERS**

VERFAHREN UND COMPUTERPROGRAMMPRODUKT ZUR STEUERUNG ANAEROBER CODIGESTER

PROCÉDÉ ET PRODUIT-PROGRAMME INFORMATIQUE PERMETTANT LA COMMANDE DE SYSTÈMES DE CO-DIGESTION ANAÉROBIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2013 ES 201331518**

(43) Date of publication of application:
**24.08.2016 Bulletin 2016/34**

(73) Proprietor: **Universidade de Santiago de Compostela**
**15782 Santiago de Compostela (ES)**

(72) Inventors:
• **LEMA RODICIO, Juan Manuel**
**E-15782 Santiago de Compostela (ES)**
• **GARCÍA GEN, Santiago**
**E-15782 Santiago de Compostela (ES)**
• **RODRÍGUEZ RODRÍGUEZ, Jorge**
**E-15782 Santiago de Compostela (ES)**

(74) Representative: **Balder IP Law, S.L.**
**Paseo de la Castellana 93**
**5ª planta**
**28046 Madrid (ES)**

(56) References cited:
**WO-A2-2011/112736    US-A1- 2008 023 397**

• **ALVAREZ J A ET AL: "A methodology for optimising feed composition for anaerobic co-digestion of agro-industrial wastes", BIORESOURCE TECHNOLOGY, ELSEVIER BV, GB, vol. 101, no. 4, 1 February 2010 (2010-02-01), pages 1153-1158, XP026737219, ISSN: 0960-8524, DOI: 10.1016/J.BIORTECH.2009.09.061 [retrieved on 2009-10-14]**
• **LETICIA REGUEIRO ET AL: "Enhanced methane production from pig manure anaerobic digestion using fish and biodiesel wastes as co-substrates", BIORESOURCE TECHNOLOGY., vol. 123, 7 August 2012 (2012-08-07), pages 507-513, XP055335076, GB ISSN: 0960-8524, DOI: 10.1016/j.biortech.2012.07.109**

- ALVAREZ, J. A. ET AL.: 'A methodology for optimizing feed composition for anaerobic co-digestion of agro-industrial wastes.' BIORESOURCE TECHNOLOGY vol. 101, no. 4, 14 October 2009, pages 1153 - 1158, XP026737219
- REGUEIRO, L. ET AL.: 'Enhanced methane production from pig manure anaerobic digestion using fish and biodiesel wastes as co-substrates.' BIORESOURCE TECHNOLOGY vol. 123, 07 August 2012, pages 507 - 513, XP055335076
- PEREZ, A. ET AL.: 'Índices of alcalinidad para el control del tratamiento anaerobio de aguas residuales fácilmente acidificables.' INGENIERÍA Y COMPETITIVIDAD vol. 10, no. 2, 2008, pages 41 - 52, XP055335127
- RODRIGUEZ, J. ET AL.: 'A hydrogen-based variable-gain controller for anaerobic digestion processses.' WATER SCIENCE AND TECHNOLOGY vol. 54, no. 2, 2006, pages 57 - 62, XP055335125
- MARTÍN-GONZÁLEZ L ET AL: "Alkalinity ratios to identify process imbalances in anaerobic digesters treating source-sorted organic fraction of municipal wastes", BIOCHEMICAL ENGINEERING JOURNAL, ELSEVIER, AMSTERDAM, NL, vol. 76, 8 April 2013 (2013-04-08), pages 1-5, XP028560641, ISSN: 1369-703X, DOI: 10.1016/J.BEJ.2013.03.016

## Description

### TECHNICAL FIELD

The invention relates to a computer-implemented control method for optimizing anaerobic digesters for the treatment of different organic waste by codigestion. The invention also relates to a computer program product for controlling anaerobic digesters appropriate to carry out the method.

The invention has application in biogas plants to improve their performance in terms of methane production, gas quality, digested product quality and operation stability. The invention allows the use of organic waste as a source of renewable energy to be optimized.

### STATE OF THE ART

The global primary energy supply for the period 2007-2030 will remain dominated by the consumption of fossil waste according to the International Energy Agency (IEA). It is estimated that fossil waste will contribute on average 77% during this period, and renewable energies will contribute 23%, and that global energy demand will continue to grow. However, it is expected that renewable energy will play a decisive role in current $CO_2$ mitigation policies. In this sense, energy from biomass and waste is positioned as one of the most important sources of renewable energy in the future. The anaerobic digestion process for treating organic waste has grown significantly in recent years, increasing the treatment capacity using this technology at rates of 25% per annum (Appels et al., 2011, Renewable and Sustainable Energy Reviews 15, pp. 4295-4301).

Anaerobic digestion is a biological process whereby any organic residue may be transformed into biogas (mixture of methane and carbon dioxide) through a complex reaction mechanism, including series and parallel reactions, which are catalyzed by different groups of microorganisms. Current advances in the design of bioreactors have increased the use of anaerobic digestion for the treatment of organic solid waste, achieving higher yields compared to conventional methods. In any case, there are many factors that can influence the performance of the digestion process, such as the type and concentration of substrate, temperature, moisture, pH, etc., (Khalid et al., 2011, 31 Waste management, 1737-1744). In this regard, anaerobic co-digestion is an interesting alternative for the improvement of the performance of anaerobic digestion of solid waste. The use of co-substrates can increase performance in biogas digesters due to the synergies that can be established between the different substrates, which allows, for example, the balance of nutrients or moisture content of the mixture to be improved, as well as being a significant economic advantage due to sharing equipment and facilities (Mata-Alvarez et al., 2000, Bioresource Technology 74, pp. 3-16).

By applying codigestion, a higher production of biogas and better quality digested product can be obtained, depending on the proportions in which the different residues are combined. Most codigestion projects that have been carried out so far are based on experimental tests of different mixtures of wastes in order to assess the viability of mixtures and biogas yield.

Alvarez, et al., 2010, Bioresource Technology 101, pp. 1153-1158 introduces an approach to the optimization of substrate mixtures for codigestion based on linear programming to optimize the mixtures. Whereas the methanogenic potential of an organic residue depends on the physicochemical characteristics of the substrate and on the concentration of the major organic components (proteins, lipids and carbohydrates), the optimization method determines the proportions of the various co-substrates that result in a higher methanogenic potential. This methodology was successfully validated by testing in batch tests.

Apart from methanogenic potential of waste, another important issue to consider during the process of anaerobic digestion is the stability of the operation. The reaction mechanism is complex because it comprises many reactions and different groups of microorganisms. As it is a biological process, the efficiency with which different microorganisms work largely depends on the conditions of pH and temperature conditions of the system. The accumulation of intermediates compounds, the presence of toxins or inhibitors can lead to acidification of the system and a loss of efficiency of the microorganisms with subsequent inactivation of these and interruption or loss of biogas production, and depending on the intensity of disturbance it can lead to an irreversible destabilization process.

The main operating parameters that are monitored in a continuous operation of an anaerobic digester are mainly: pH, alkalinity, concentration of volatile fatty acids (VFA), and the flow rate and composition of biogas. However, the response and sensitivity of these parameters to a disturbance are different. Boe et al., 2010, Water Research 44, 5973-5980 studied the response of these process indicators by exposing them to different types of disturbances in order to find the most suitable parameter for process monitoring. In particular, they studied the behavior of the following indicators: biogas production, pH, dissolved VFA and Hydrogen (measured on-line), and methane and hydrogen content in the biogas (measured off-line). They noted that biogas production increased by increasing the organic load but with a slight decrease in methane content. The pH remained relatively stable and did not show a clear response to hydraulic overload, but it was sensitive to organic overloads. The dissolved hydrogen was very sensitive to the presence of easily biodegradable compounds, and hydrogen in gas phase was increased after an organic overload. They found that the concentration of VFA was an effective indicator, where propionate was the most persistent acid after occurrence of the disturbance.

The most important aspects to take into account by operators in biogas plants to monitor their operation are robustness, simplicity, precision, accuracy and reliability of the most important parameters of the process. Small anaerobic digestion plants, especially mono-digestion plants, can be controlled in an acceptable manner using simple valuation methods. However, centralized biogas plants that operate using codigestion require a more reliable and comprehensive monitoring. The simple measurement of acidity or any other parameter is probably not enough to reveal the true state of the process due to the variability of the supply mixture. Moreover, the control of raw materials should be another part of the analysis process. Current advances in process analysis technology, which develop new spectroscopic and electrochemical methods of measurement, together with multivariable data analysis, offer new possibilities for monitoring the anaerobic digestion process that allow a more effective and reliable control of the process (Madsen et al., 2011, Renewable and Sustainable Energy Reviews 15, pp. 3141-3155).

## DESCRIPTION OF THE INVENTION

The invention relates to a computer-implemented control method for the optimization of anaerobic digesters in terms of methane production. In one aspect, the invention relates to a method that controls anaerobic codigestion operations simultaneously treating a mixture of two or more organic waste by anaerobic digestion. The supply to the digester comprises agro-industrial organic wastes such as pig slurry, cow slurry, chicken slurry, waste from biodiesel production industry, distillery residues or residues of the canning industry.

The method comprises the following steps:

a. determination through linear programming techniques the proportion of the different waste substrates of the mixture and the hydraulic residence time of the system (HRT) by maximizing an objective function, and thus, methane production, subject to linear restrictions;

b. diagnosis of the co-digestion plant stability by monitoring physical and chemical parameters, comprising the alkalinity ratio, considered as the ratio of alkalinity due to volatile fatty acids and alkalinity due to bicarbonate, and the methane flow prate of the anaerobic digestion process, and the calculation of diagnosis parameters indicative of the stability of the system, comprising a stability factor, determined according to the alkalinity ratio, and remaining methanogenic capacity factor, determined from the methane flow rate;

c. control action, which produces the modification of the limits of the linear restrictions of the linear programming method, step a, from the parameters provided in the diagnosis step that modifies the proportion and flow rate of the waste substrates of the mixture in order to maximize methane production or restore operation stability, maintaining the quality of biogas and digested product in the margins set by the restrictions; and

d. repeating steps a) to c), wherein the determination of the objective function and the parameters used in the characterization of the mixture referred to in the objective function are as specified in the claims and here below.

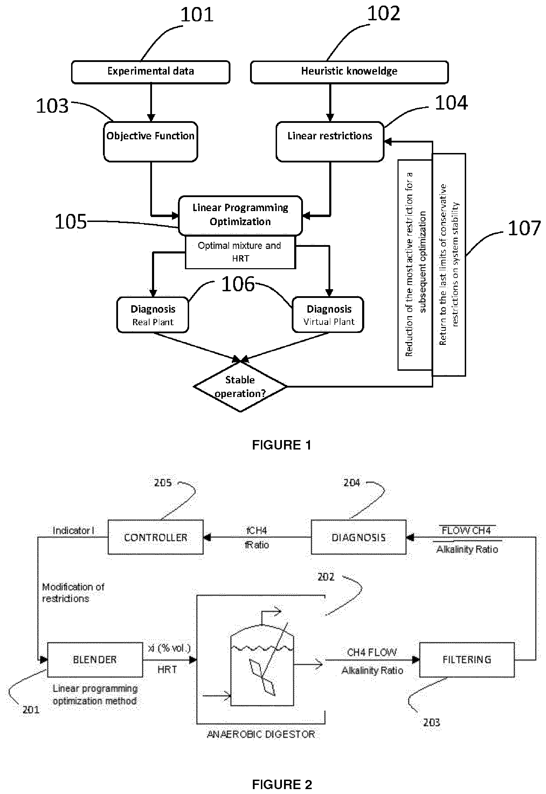

Figure 1 shows a particular embodiment of the method, in which the various blocks thereof are shown. Both the experimental data (101), comprising the composition and methanogenic potential of each substrate, including heuristic knowledge of anaerobic digestion processes (102), which allows the operation restrictions that the feed mixture to the reactor must meet to be set, are the basis to posing and solving the optimization problem using linear programming (105). The optimization of an objective function (103), in order to maximize the methane production and considering the restrictions applied to the mixtures (104), determines the operating conditions of the reactor (in terms of mixture of substrates and HRT system). These conditions are maintained for a certain time (controller cycle time), after which an assessment of the stability and performance of the process (106) is carried out. The results of the diagnosis determine to what extent operational supply restrictions need to be modified to more or less conservative scenarios (107). Lastly, the control action modifies the limits of linear restrictions (104) allowing a new supply mixture to the reactor to be calculated.

The computer-implemented control method enables the application thereof both to a real anaerobic co-digestion plant and to a simulated plant containing a sufficiently accurate model such as the Anaerobic Digestion Model No. 1 (ADM1), developed by the International Water Association (Batstone et al., 2002, Water Science and Technology 45, pp. 65-73).

The computer-implemented control method can be grouped into the following modules: linear programming or *Blender* module (201), *Filter* module (203), *Diagnosis* module (204) and *Controller* module (205). Thus, in a first stage in the computer-implemented control method, the linear programming module (201) that returns the optimum reactor feed mixture and the hydraulic residence time (HRT) of the system is run. The HRT and the mixture of optimal substrates obtained in the *Blender* module are applied in the real/simulated plant (202). The *Filtering* module calculates the average value of the physical and chemical parameters to be considered in the *Diagnosis* module for each control cycle (1/4 HRT), which are alkalinity ratio (Ratio) and methane flow (QCH4). The stability and performance of the operation of the digester is continuously monitored by specific indicators defined in the *Diagnosis* module (204). The *Controller* module (205) calculates an indicator at the end of each controller cycle time (which is set in a quarter of time of the HRT system) and acts on the most active restriction optimization problem (one that has the potential to achieve a further increase in the value of the objective function). A restriction is activated when the solution of linear programming is in the upper or lower limit of that particular restriction. In the case of there being more than one active restriction, it is considered that the most active restriction is that which involves a further increase in the value of the objective function. Lastly and closing the loop of the control procedure, the Blender module calculates a new HRT and a new mix of substrates, initiating a new cycle control with the new limits of operational restrictions. In a particular embodiment, the system is implemented using MATLAB® / Simulink. The different modules of the computer-implemented control method are now described in detail.

### Blender

The optimization method using linear programming applied in the *Blender* module simultaneously leads to the optimization of the substrate mixture and the HRT of the system that maximize the objective function. For each value of HRT evaluated, a substrate mixture is obtained. The combination of HRT and substrate mixture which produces a higher methane production, considering the restrictions applied, is returned as an optimal operation.

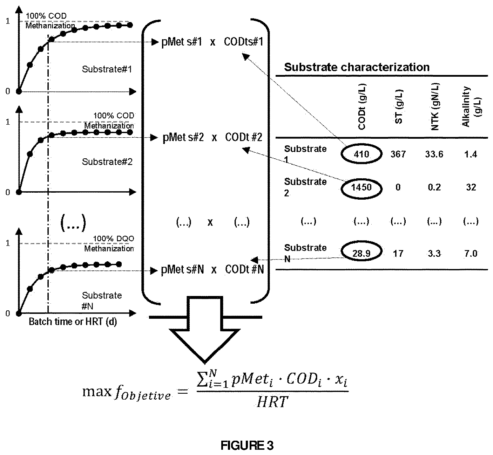

The structure of the objective function ($f_{Objective}$) of the optimization problem is presented in Figure 3, and has the form:

$$f_{Objective} = \frac{\sum_{i=1}^{N} pMet_i \cdot CODt_i \cdot x_i}{HRT}$$

wherein $x_i$ is the volume fraction of each waste substrate in the mixture; $CODt_i$ is the chemical oxygen demand, indicative of the organic content of each waste substrate (obtained from the substrate characterization table); $pMet_i$ is the percentage of methanation expected for a given HRT, which is obtained experimentally from biodegradability tests of each waste substrate and expressed as a decimal, and HRT is the hydraulic residence time to be applied to the system and has been obtained from biodegradability tests of all waste substrates of the mixture. The objective function returns the sum of the terms obtained by multiplying the fraction of each substrate in the mixture, xi by its $CODt_i$ and the expected percentage of methanogenic conversion ($pMet_i$) for the given HRT (taken from degradation test). The units of the objective function are g COD-CH$_4$ / L ·d. Methane production, despite being usually expressed in L CH$_4$ / L ·d (or m$^3$ CH$_4$ / m$^3$ ·d), is expressed in this case in g COD / L ·d, since it allows for a direct comparison with the organic loading rate (OLR) of the system, which is expressed in the same units and is one of the main operating parameters in anaerobic digestion together with HRT. In this way, the efficiency of methanation with respect to the organic load that is supplied to the digester can be quickly calculated. The definition of the objective function uses the experimental information from each biodegradability test of the discontinuous substrates as well as the content of organic matter (COD) thereof. These tests are performed routinely in many anaerobic digestion installations and therefore do not entail an additional requirement when implementing this control strategy. Likewise, the total COD value of the substrates is typically a known variable. Thus, a direct application system is achieved even in installations with limited analytical instrumentation and capacity.

In addition to the objective function, the optimization problem provides a set of linear restrictions based on the operating experience of anaerobic digestion. Linear restrictions for the supply are defined based on the available characterization of substrates. Restrictions begin with very conservative values of anaerobic digestion processes. Maximum and minimum acceptable values for various parameters are defined, among others: (1) organic loading rate (OLR); (2) Total Kjeldahl Nitrogen (TKN); (3) Humidity; (4) lipid content; (5) total alkalinity; (6) salinity (chlorides), (7) quality of biogas; and (8) digested product quality. These ranges are formulated as linear restrictions of the objective function to optimize and ensure that the waste substrate mixture is calculated within these limits and is therefore operationally feasible.

Specifically, the OLR restrictions (1), (7) quality of biogas and (8) digested product quality are not characteristics obtained directly from the characterization of the waste substrates. The OLR of each waste substrate was calculated from the CODt of each waste substrate and HRT of the system being evaluated, and the quality of the digested product from the biodegradable COD of each waste substrate. Their expressions are: OLR = COD$_t$/ HRT (expressed in g COD/L ·d)

Quality of digested product = COD$_t$ (pBiod-pMet) (expressed in g COD/L) wherein pBiod is the maximum percentage of methanation (or % biodegradability, expressed in %/100) obtained from the biodegradability tests of each substrate; and pMET is the percentage of methanation (in %/100) reached at the moment of time of the biodegradability curve equal to the HRT applied to the digester.

The gas quality is calculated in terms of the maximum sulfur content (expressed in g $H_2S$ / L) that can be obtained from the biogas. It is calculated from the mass balance of the sulfur considering that all the sulfur of the substrates is converted to $H_2S$ and is completely eliminated in the gas stream:

Balance: $\sum xi \cdot [H_2S]i \cdot Q_e = Q_g \cdot [H_2S]_g$
Quality of the gas = $[H_2S] \cdot Q_e / Q_g$

wherein $Q_e$ is the flow rate into the digester (L / d); $Q_g$ is the expected flow rate of biogas to the OLR of the system (calculated as $Q_g = OLR \cdot V \cdot (0.35/0.70)$, where V is the volume of the digester (in L), 0.35 is the ratio L $CH_4$ / g COD obtained from the complete oxidation of the methane at normal conditions, and 0.70 refers to the typical value of methane composition that is produced in anaerobic digestion (expressed in %/100); xi is the volume fraction of each substrate in the mixture. Finally, the term $[H_2S] \cdot Qe/Qg$ represents quality of the gas associated with each substrate.

**Filtering**

In this module, the average value of the physical and chemical parameters to be considered in the *Diagnosis* module for each control cycle, which are alkalinity ratio (Ratio) and methane flow rate ($Q_{CH4}$), is calculated. The average values of these parameters in each control cycle values are compared in the diagnosis module with reference values or a baseline or reference for evaluating the operation stability. The filtered values or average values of these parameters are calculated by means of the following expressions:

$$\overline{Ratio} = \frac{\int Ratio(t)\, dt}{\int dt}$$

$$\overline{Q_{CH4}} = \frac{\int Q_{CH4}(t)\, dt}{\int dt}$$

wherein *Ratio* is the relationship of average alkalinities *and* $\overline{Q_{CH4}}$ is the average methane flow rate. The integration time used is equal to one fourth of the HRT.

**Diagnosis**

The *control system Diagnosis* module of the performance and stability of the control system monitors the parameters *Average Alkalinity Ratio* and *Average Methane Flow rate*. The Alkalinity Ratio parameter is defined as the ratio of alkalinity due to volatile fatty acids (VFA) and alkalinity due to bicarbonate, and allows organic acidification and system overload to be predicted. The average methane flow rate is used as an indicator of the proximity of operation to the maximum capacity of the process.

The *Diagnosis* module returns two factors based on the representative values of Average *Alkalinity Ratio* and Average *Methane* flow rate, which are the stability factor ($f_{Ratio}$) and remaining methanogenic capacity factor ($f_{CH4}$).

The stability factor ($f_{Ratio}$) is calculated using an empirical correlation (Rodriguez et al., 2006, Water Science & Technology 54, pp. 57-62) based on the *average Ratio*, $\overline{Ratio}$, with respect to the reference value *Ratio\** set as a limit of system stability. The returned value is in the range [-1, 1] and serves as an indicator of process instability when negative, and as level of stability when it is positive. The stability factor is calculated using the following expression:

$$f_{Ratio} = \begin{cases} \left( 1 - \dfrac{\overline{Ratio}}{Ratio\,*} \right)^{\frac{1}{m}} & if \;\; \overline{Ratio} \leq Ratio\,* \\[4mm] \left( \dfrac{Ratio\,*}{\overline{Ratio}} \right)^{n} - 1 & if \;\; \overline{Ratio} > Ratio\,* \end{cases}$$

wherein m and n are values empirically determined from experimental data fit.

The Remaining Methanogenic Potential Factor ($f_{CH4}$) is calculated using an empirical correlation based on the average methane production, $\overline{Q_{CH4}}$, with respect to a reference value of methane flow established as maximum system capacity, $\overline{Q_{CH4}}\,*$ (Rodriguez et al., 2006, Water Science & Technology 54, pp. 57-62). The result is comprised between [0,1] and serves to moderate the extent to which the changes of organic load applied to the system, avoiding for example, large increases in very stable conditions when methane production is close to that which is estimated as maximum system capacity. The remaining potential methanogenic potential factor ($f_{CH4}$) is calculated using the following expression:

$$f_{CH4} = \frac{\alpha \cdot Q^{*}_{CH4}}{\overline{Q_{CH4} + \alpha \cdot Q^{*}_{CH4}}}$$

wherein $\alpha$ is empirically determined from experimental data fit.

**Controller (Linear Restriction Adaption)**

The *Controller* module calculates a control indicator, indicator I, at the end of each time cycle which is the product of $f_{Ratio}$ and $f_{CH4}$ factors when the system is stable (and therefore $f_{Ratio}$ is positive), and equal to $f_{Ratio}$ when the system is not stable (and therefore $f_{Ratio}$ is negative).

$$Indicator\;\; I = \begin{cases} f_{Ratio} \cdot f_{CH4} & if \;\; f_{Ratio} > 0 \\[2mm] f_{Ratio} & if \;\; f_{Ratio} \leq 0 \end{cases}$$

The value of the indicator I is in the range [-1,1]. The control action produces the modification of the limits of operational restrictions of linear optimization problem. The value of the indicator I is applied directly to the most active linear restriction resulting from solving the linear programming problem. When a supply mixture to the reactor is calculated for each control cycle, the solution of the linear programming returns the active restrictions of the system, which would allow the value of the objective function to be increased if the limits of these restrictions were to be extended.

The control action modifies the limit of the most active restriction through an equation in which the new limit calculated takes into account the current value of the restriction limit, the range of the restriction (difference between the upper and lower limit restriction) and indicator I.

$$\mathrm{Limit}_{NEW} = Limit_{CURRENT} + indicator\;I \cdot \left( Limit_{UP} - Limit_{DOWN} \right)$$

wherein $Limit_{NEw}$ is the new limit, $Limit_{CURRENT}$ the current limit and $Limit_{UP}$ and $Limit_{DOWN}$ are respectively the upper and lower limits of the range. When the system is stable, modifying the ratio of the components of the mixture in order to maximize methane production comprises increasing the range (difference between the upper and lower limit) of the most active restriction of the set of restrictions of the linear programming problem in order to increase the organic load treated in the reactor and favor the system increasing the methane production. The control indicator *I*, comprised between [0, 1] when the system is stable, means that the limit of the most active restriction is modified and increases the range of values for that parameter. As a result of these new limits, the solution of the linear programming problem will be different and it will promote a mixture with greater methane potential production, and therefore the system will work at a faster organic load velocity. If the limits of the restrictions imposed in the linear programming module are increased, which restricts the value of the objective function, the system will be able to work with a mixture and HRT which allows for obtaining a higher methane production and therefore the system operates at a greater OLR.

On the other hand, the rate of conversion of organic matter to methane will be that which allows for meeting the effluent quality in terms of COD, in other words, without exceeding the maximum considered in the restriction concerning the quality of the digested product. Thus, the system will tend to maximize the methane production and simultaneously obtain a degree of conversion enabling the quality restriction of the digested product to be met.

In destabilization episodes, the goal is to reduce the organic load treated in the reactor and encourage the system to recover from the perturbation. In these cases, the control indicator I will take a value between [-1 , 0], causing the modification of the limit of the more active restriction, thus reducing the range of values for that parameter. As a result of these new limits, the solution of the linear programming problem will be different, reducing the value of the objective function, working to lower organic loads and favoring the recovery of the system from destabilization.

According to another aspect, the invention provides a computer program product comprising program instructions for causing a computer system to perform the method and process the information concerning the composition of the mixture of organic waste mixture.

Said computer program may be stored in a physical storage media such as a recording means, a computer memory or a read-only memory, or may be carried by a carrier wave, such as electrical or optical.

## BRIEF DESCRIPTION OF THE FIGURES

The embodiments detailed in the figures are illustrated by way of example and not by way of limitation:

Figure 1 shows a block diagram of the computer-implemented control method for anaerobic codigestion of multiple substrates in which optimization is achieved by applying a control method based on linear programming to control the mixture of substrates, diagnosis of process performance and feedback to the operating restrictions.

Figure 2 shows the computer-implemented control method of an anaerobic co-digestion plant in which all blocks are involved.

Figure 3 shows that the objective function is evaluated for a given set of HRT and a set of substrate fractions in the mixture (xi).

Figure 4 shows the optimization results of the slurry, glycerin and gelatin codigestion simulation.

a) Percentage of substrates in the mixture (% COD) together with the applied OLR;
b) Stability factors

($f_{Ratio}$) and remaining methanogenic potential ($f_{CH4}$) and control indicator I; c) Ratio of alkalinity; d) Methane production and applied OLR.

## EXAMPLES

The computer-implemented control method of the present invention has been tested in simulations connected to a virtual anaerobic co-digestion plant. The virtual plant consists of an anaerobic co-digestion model implemented in MATLAB® / Simulink® and it allows processes in continuous and discontinuous operation to be simulated (Garcia-Gen et al., 2013, Bioresource Technology 147, pp. 525-533). The model is based on a modification of Anaerobic Digestion Model No. 1 (Batstone et al., 2002, Water Science and Technology 45, pp. 65-73).

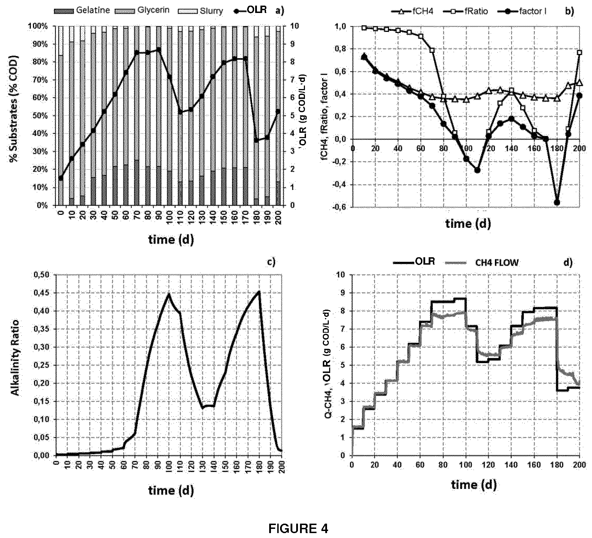

A simulation of 200 days of continuous codigestion operation with the mixture of three substrates (pig slurry, biodiesel waste (glycerin) and gelatin) was carried out in an Upflow Anaerobic Sludge Blanket UASB type digester of 1 $m^3$ useful volume using the virtual plant connected to the closed loop control strategy. The results obtained are shown in Figure 4.

The results show that the computer-implemented control method leads to an automatic optimization of the mixture and HRT. During operation, the HRT of the system ranged between 32-40 days, the average value for the entire operation being 39 days. Moreover, it is observed that the composition of the supply mixture and the organic loading rate (OLR) were modified during operation due to the control action. The cycle time of the controller was set at 10 days (one quarter HRT time) a time interval in which the diagnosis of the digester and stability control action is performed.

The system started operation at a low OLR of 1.5 g COD / L d and reached values of 8.5 g COD / L d after about 70 days. During the first 90 days, the controller increased the OLR of the system, thus promoting an increase in methane production. During days 90-110, as a result of the increased diagnostic parameter, alkalinity ratio above the stability threshold (the setpoint value of which was set at 0.4), the control action reduced the OLR during the time interval [100,120) to counteract the destabilization of the system and lead the digester to a safer operating state. With this action, the controller was able to bring the system to a more stable operation, reducing and moving the diagnosis parameter away from the destabilization threshold.

Once the system had stabilized between days 110-120, the control system began to increase the OLR again until the process reached higher alkalinity ratio values than the setpoint. At that point, the control action works to reduce the increasing destabilization of the system, and as before, the OLR is reduced until the alkalinity ratio is at values away from the stability limit.

During optimization of the operation, the limits of the linear restrictions that were applied to the linear programming problem for the calculation of the optimal mixture were modified (increasing or reducing) in order to obtain an optimal mixture that maximizes the methane production for a given OLR or to obtain a mixture that would lead to a more stable operation, but also where methane production was the maximum possible given those conditions. The initial limits of the restrictions were established according to typical operating ranges in co-digestion plants. During the optimization of this operation OLR, NTK, and total alkalinity restriction limits were modified.

In this example, for a continuous operation of anaerobic co-digestion, it has been demonstrated that the control strategy developed has worked well for 200 days in closed loop operation. The control system response has been effective in both directions, both to optimize the supply mixture to the reactor and to obtain the maximum methane production when the system is stable, as to bring the digester to a state of safe operation when a destabilization has taken place. The limits of the restrictions were extended or reduced depending on system stability, obtaining in each case the optimal mixture that maximized the production of methane.

## Claims

1. A computer-implemented method for controlling anaerobic codigestion plants of waste mixtures comprising the following steps:

   a. determination through linear programming techniques of the proportion of the different waste substrates of the mixture and the hydraulic residence time of the system (HRT) that maximize an objective function, and thus, methane production, subject to linear restrictions;
   b. diagnosis of the codigestion plant stability by monitoring physical and chemical parameters, comprising the alkalinity ratio, considered as the ratio of alkalinity due to volatile fatty acids and alkalinity due to bicarbonate, and the methane flow rate of the anaerobic digestion process, and the calculation of diagnosis parameters indicative of the stability of the system, comprising a stability factor, determined according to the alkalinity ratio, and a factor of remaining methanogenic capacity, determined from the methane flow rate;
   c. control action, which produces the modification of the limits of the linear restrictions of the linear programming method, step a of the method, from the parameters provided in the diagnosis step, that modifies the proportion and flow rate of the waste substrates of the mixture in order to maximize the methane production or restore operation stability, maintaining the quality of the digested product and the biogas, in the margins set by the restrictions; and
   d. repeat steps a) to c); wherein

   (i) the determination of the ratio of the waste substrates of the mixture and the HRT comprises determining an objective function ($f_{Objective}$)

$$f_{Objective} = \frac{\sum_{i=1}^{N} pMet_i \cdot CODt_i \cdot x_i}{HRT}$$

   wherein $x_i$ is the volume fraction of each waste substrate in the mixture; $CODt_i$ is the organic matter content of each waste substrate , i.e. the chemical oxygen demand; $pMet_i$ is the percentage of methanation expected for the given HRT, taken from biodegradability tests of each waste substrate and expressed as a decimal, and HRT is the hydraulic residence time to be applied to the system and has been obtained from biodegradability tests of all waste substrates of the mixture, and
   (ii) the parameters used in the characterization of the mixture referred to in the objective function, and which are the basis for defining the linear restrictions, include, among others, organic loading rate (OLR), Total Kjeldahl Nitrogen (TKN), moisture, lipid content, total alkalinity, chloride content, biogas quality and digested product quality.

2. The method according to claim 1, wherein the waste comprises pig slurry waste, cow slurry waste, chicken slurry waste, waste from biodiesel production industry, distillery residues or residues of the canning industry.

3. The method according to claim 1, wherein the alkalinity ratio and methane flow rate parameters are averaged in a filtering step prior to the diagnosis step, according to the following expressions

$$\overline{Ratio} = \frac{\int Ratio(t)\, dt}{\int dt}$$

$$\overline{Q_{CH4}} = \frac{\int Q_{CH4}(t)\, dt}{\int dt}$$

wherein $\overline{Ratio}$ is the average alkalinity ratio and $\overline{Q_{CH4}}$ is the average methane flow rate.

4. The method according to claim 3, **characterized in that** the average time to perform the integration is preferably ¼HRT.

5. The method according to claim 1, wherein the parameters indicative of stability comprise the stability factor ($f_{Ratio}$), the remaining methanogenic capacity factor ($f_{CH4}$) and control indicator I, according to the following expressions:

$$f_{Ratio} = \begin{cases} \left(1 - \dfrac{\overline{Ratio}}{Ratio*}\right)^{\frac{1}{m}} & if \ \overline{Ratio} \le Ratio* \\[2em] \left(\dfrac{Ratio*}{\overline{Ratio}}\right)^{n} - 1 & if \ \overline{Ratio} > Ratio* \end{cases}$$

$$f_{CH4} = \frac{\alpha \cdot Q_{CH4}^{*}}{\overline{Q_{CH4}} + \alpha \cdot Q_{CH4}^{*}}$$

$$Indicator \ I = \begin{cases} f_{Ratio} \cdot f_{CH4} & if \quad f_{Ratio} > 0 \\ f_{Ratio} & if \quad f_{Ratio} \le 0 \end{cases}$$

wherein *Ratio** and Q*$_{CH4}$ are the reference values of the alkalinity ratio and methane flow rate, respectively and m, n and $\alpha$ are parameters empirically obtained from experimental data fit.

6. The method according to claim 5, **characterized in that** if the indicator value I is negative, the system is destabilized and proceeds to restrict the range of the most active restriction, which decreases the objective function; whereas if the value of the indicator I is positive, the system is stable and the limits of the most active restriction are increased obtaining a higher value of the objective function.

7. The method according to claim 6, **characterized in that** the control action modifies the limit of the most active restriction through an equation in which the new limit is calculated taking into account the current value of the restriction limit, the range of the restriction, difference between the upper and lower limit of the restriction, and the indicator I according to the following expression:

$$\text{Limit}_{NEW} = Limit_{CURRENT} + indicator\,l \cdot \left(Limit_{UP} - Limit_{DOWN}\right)$$

wherein $Limit_{NEW}$ is the new limit, $Limit_{CURRENT}$ is the current limit and $Limit_{UP}$ and $Limit_{DOWN}$ are, respectively, the upper and lower limits of the range.

**8.** The method according to claims 6 to 7, **characterized in that** the modification of the components of the mixture is carried out every quarter of the hydraulic residence time (HRT).

**9.** A computer program product comprising program instructions for causing a computer system to perform the method for controlling an anaerobic codigestion plant according to any one of claims 1 to 8.

**10.** A computer-readable storage means having stored thereon the computer program product of claim 9.

**11.** A data carrier wave carrying the computer program product of claim 9.

**Patentansprüche**

**1.** Computerimplementiertes Verfahren zum Steuern anaerober Ko-Vergärungsanlagen von Abfallgemischen mit den folgenden Verfahrensschritten:

a. Bestimmung, mittels linearer Programmiertechniken, des Verhältnisses der verschiedenen Abfallsubstrate des Gemisches und der hydraulischen Verweilzeit des Systems (HRT), welche eine Zielfunktion und damit die Methanproduktion vorbehaltlich linearer Beschränkungen maximieren;
b. Diagnose der Stabilität der Ko-Vergärungsanlage, indem physikalische und chemische Parameter überwacht werden, einschließlich des Alkalinitätsverhältnisses, welches als das Verhältnis von auf flüchtige Fettsäuren zurückzuführender Alkalinität und von auf Bikarbonat zurückzuführender Alkalinität angesehen wird, und der Methan-Durchflussrate des anaeroben Vergärungsprozesses, und der Berechnung von Diagnoseparametern, die die Stabilität des Systems anzeigen, einschließlich eines Stabilitätsfaktors, der entsprechend dem Alkalinitätsverhältnis bestimmt wird, und einem Faktor verbleibender methanogener Kapazität, welcher aus der Methan-Durchflussrate bestimmt wird;
c. Steueraktion, welche die Modifikation der Begrenzungen der linearen Beschränkungen des linearen Programmierverfahrens, Schritt a des Verfahrens, von den in dem Diagnoseschritt bereitgestellten Parametern bewirkt, welche das Verhältnis und die Durchflussrate der Abfallsubstrate des Gemisches verändert, um die Methanproduktion zu maximieren oder die Betriebsstabilität wieder herzustellen, wobei die Qualität des vergorenen Produkts und des Biogases innerhalb der von den Beschränkungen gesetzten Grenzen beibehalten wird; und
d. Wiederholen der Schritte a) bis c); wobei

(i) die Bestimmung des Verhältnisses der Abfallsubstrate des Gemischs und des HRT das Bestimmen einer Zielfunktion ($f_{Objective}$) beinhaltet

$$f_{Objective} = \frac{\sum_{i=1}^{N} pMet_i \cdot CODt_i \cdot x_i}{HRT}$$

wobei $x_i$ der Volumenanteil jedes Abfallsubstrats in dem Gemisch ist; $CODt_i$ der Gehalt an organischer Substanz jedes Abfallsubstrats, d.h. der chemische Sauerstoffbedarf, ist; $pMeti$ der Prozentsatz der für das gegebene HRT erwarteten Methanisierung ist, welcher von Tests der biologischen Abbaubarkeit jedes Abfallsubstrats genommen und als eine Dezimale ausgedrückt wird, und HRT ist die auf das System anzuwendende hydraulische Verweilzeit, und wurde aus Tests der biologischen Abbaubarkeit aller Abfallsubstrate des Gemischs erhalten, und
(ii) die für die Charakterisierung des Gemischs der in der Zielfunktion verwendeten Parameter, und die die Grundlage zum Bestimmen der linearen Beschränkungen sind, unter anderem organische Beladungsrate (OLR), Gesamter Kjeldahl-Stickstoff (TKN), Feuchtigkeit, Lipidgehalt, Gesamtalkalinität, Chloridgehalt, Qualität des Biogases und Qualität des vergorenen Produkts beinhalten.

2. Verfahren nach Anspruch 1, bei dem der Abfall Schweinegülleabfälle, Kuhmistabfälle, Hühnermistabfälle, Abfälle der Biodiesel produzierenden Industrie, Brennereirückstände oder Rückstände der Konservenindustrie beinhaltet.

3. Verfahren nach Anspruch 1, bei dem die Alkalinitätsverhältnis- und Methan-Durchflussrate-Parameter vor dem Diagnoseschritt in einem Filterschritt nach den folgenden Ausdrücken gemittelt werden

$$\overline{Ratio} = \frac{\int Ratio(t)\, dt}{\int dt}$$

$$\overline{Q_{CH4}} = \frac{\int Q_{CH4}(t)\, dt}{\int dt}$$

wobei *Ratio* das durchschnittliche Alkalinitätsverhältnis und $\overline{Q_{CH4}}$ die durchschnittliche Methan-Durchflussrate ist.

4. Verfahren nach Anspruch 3, **dadurch gekennzeichnet, dass** die Durchschnittszeit, um die Integration durchzuführen, vorzugsweise ¼HRT ist.

5. Verfahren nach Anspruch 1, bei dem die die Stabilität anzeigenden Parameter den Stabilitätsfaktor ($f_{Ratio}$), den Faktor verbleibender methanogener Kapazität ($f_{CH4}$) und einen Steuerindikator I nach den folgenden Ausdrücken umfassen:

$$f_{Ratio} = \begin{cases} \left(1 - \dfrac{\overline{Ratio}}{Ratio*}\right)^{\frac{1}{m}} & \textit{wenn}\ \ \overline{Ratio} \leq Ratio* \\[4mm] \left(\dfrac{Ratio*}{\overline{Ratio}}\right)^{n} - 1 & \textit{wenn}\ \ \overline{Ratio} > Ratio* \end{cases}$$

$$f_{CH4} = \frac{\alpha \cdot Q_{CH4}^{*}}{Q_{CH4} + \alpha \cdot Q_{CH4}^{*}}$$

$$Indicator\ I = \begin{cases} f_{Ratio} \cdot f_{CH4} & \textit{wenn}\ \ f_{Ratio} > 0 \\[2mm] f_{Ratio} & \textit{wenn}\ \ f_{Ratio} \leq 0 \end{cases}$$

wobei *Ratio** und $Q_{CH4}^{*}$ jeweils die Referenzwerte des Alkalinitätsverhältnisses und der Methan-Durchflussrate sind und m, n und $\alpha$ Parameter, die empirisch aus der Anpassung experimenteller Daten gewonnen wurden.

6. Verfahren nach Anspruch 5, **dadurch gekennzeichnet, dass** das System, wenn der Indikatorwert I negativ ist, destabilisiert ist und damit fortfährt, den Bereich der aktivsten Beschränkung zu beschränken, was die Zielfunktion vermindert; während das System, wenn der Indikatorwert I positiv ist, stabil ist und die Grenzen der aktivsten Beschränkung erhöht werden, was zu einem höheren Wert der Zielfunktion führt.

7. Verfahren nach Anspruch 6, **dadurch gekennzeichnet, dass** die Steueraktion die Grenze der aktivsten Beschränkung durch eine Gleichung verändert, in der die neue Grenze unter Berücksichtigung des aktuellen Wertes der Beschränkungsgrenze, des Bereichs der Beschränkung, der Differenz zwischen der oberen und der unteren Grenze der Beschränkung, und des Indikators I gemäß dem folgenden Ausdruck berechnet wird:

$$\text{Limit}_{NEW} = Limit_{CURRENT} + indicator\ I \cdot \left( Limit_{UP} - Limit_{DOWN} \right)$$

wobei LimitNEw die neue Grenze ist, $Limit_{CURRENT}$ die aktuelle Grenze und $Limit_{UP}$ und $Limit_{DOWN}$ jeweils die oberen und unteren Grenzen des Bereichs sind.

8. Verfahren nach Anspruch 6 bis 7, **dadurch gekennzeichnet, dass** die Veränderung der Komponenten des Gemischs zu jedem Viertel der hydraulischen Verweilzeit (HRT) durchgeführt wird.

9. Computer-Programmprodukt mit Programmanweisungen, um zu bewirken, dass ein Computersystem das Verfahren zum Steuern einer anaeroben Ko-Vergärungsanlage nach einem beliebigen der Ansprüche 1 bis 8 durchführt.

10. Computerlesbares Speichermittel, auf dem das Computer-Programmprodukt nach Anspruch 9 gespeichert ist.

11. Datenträgerwelle, welche das Computer-Programmprodukt nach Anspruch 9 trägt.

**Revendications**

1. Procédé mis en œuvre par ordinateur pour commander des installations de codigestion anaérobie de mélanges de déchets comprenant les étapes suivantes :

   a. la détermination par des techniques de programmation linéaire de la proportion des différents substrats de déchets du mélange et du temps de séjour hydraulique du système (HRT) qui maximisent une fonction d'objectif, et donc, la production de méthane, soumise à des restrictions linéaires ;
   b. le diagnostic de la stabilité de l'installation de codigestion par la surveillance de paramètres physiques et chimiques, comprenant le rapport d'alcalinité, considéré comme étant le rapport entre l'alcalinité due aux acides gras volatils et l'alcalinité due au bicarbonate, et le débit de méthane du processus de digestion anaérobie, et le calcul de paramètres de diagnostic indicatifs de la stabilité du système, comprenant un facteur de stabilité, déterminé en fonction du rapport d'alcalinité, et un facteur de capacité méthanogène restante, déterminé à partir du débit de méthane ;
   c. action de contrôle, qui produit la modification des limites des restrictions linéaires de la méthode de programmation linéaire, étape a de la méthode, à partir des paramètres fournis dans l'étape de diagnostic, qui modifie la proportion et le débit des substrats de déchets du mélange afin de maximiser la production de méthane ou de restaurer la stabilité du fonctionnement, en maintenant la qualité du produit digéré et du biogaz, dans les marges fixées par les restrictions ; et
   d. répéter les étapes a) à c) ; dans lequel

   la détermination du rapport entre les substrats de déchets du mélange et le HRT comprend la détermination d'une fonction d'objectif ($f_{Objective}$)

$$f_{Objective} = \frac{\sum_{i=1}^{N} pMet_i \cdot CODt_i \cdot x_i}{HRT}$$

où $xi$ est la fraction volumique de chaque substrat de déchets dans le mélange ;

*CODti* est la teneur en matière organique de chaque substrat de déchets, c'est-à-dire la demande chimique en oxygène ;

$pMet_i$ est le pourcentage de méthanisation attendu pour le HRT donné, pris à partir de tests de biodégradabilité de chaque substrat de déchets et exprimé sous forme décimale, et HRT est le temps de séjour hydraulique à appliquer au système et qui a été obtenu à partir de tests de biodégradabilité de tous les substrats de déchets du mélange, et

(ii) les paramètres utilisés dans la caractérisation du mélange auxquels il est fait référence dans la fonction d'objectif, et qui sont à la base de la définition des restrictions linéaires, comprennent, entre autres, le taux de charge organique (OLR), l'azote Kjeldahl total (TKN), l'humidité, la teneur en lipides, l'alcalinité totale, la teneur en chlorure, la qualité du biogaz et la qualité du produit digéré.

2. Procédé selon la revendication 1, dans lequel les déchets comprennent des déchets de lisier de porc, des déchets de lisier de vache, des déchets de lisier de poulet, des déchets de l'industrie de production de biodiesel, des résidus de distillerie ou des résidus de l'industrie de la conserverie.

3. Procédé selon la revendication 1, dans lequel les paramètres de rapport d'alcalinité et de débit de méthane sont soumis à un calcul de moyenne dans une étape de filtrage précédant l'étape de diagnostic, selon les expressions suivantes

$$\overline{Ratio} = \frac{\int Ratio(t)\,dt}{\int dt}$$

$$\overline{Q_{CH4}} = \frac{\int Q_{CH4}(t)\,dt}{\int dt}$$

où *Ratio* est le taux d'alcalinité moyen et $\overline{Q_{CH4}}$ est le débit de méthane moyen.

4. Procédé selon la revendication 3, **caractérisé en ce que** le temps moyen pour réaliser l'intégration est de préférence ¼HRT.

5. Procédé selon la revendication 1, dans lequel les paramètres indicatifs de stabilité comprennent le facteur de stabilité ($f_{Ratio}$), le facteur de capacité méthanogène restante ($f_{CH4}$) et l'indicateur de contrôle I, selon les expressions suivantes :

$$f_{Ratio} = \begin{cases} \left(1 - \dfrac{\overline{Ratio}}{Ratio\,*}\right)^{\frac{1}{m}} & if\ \overline{Ratio} \leq Ratio\,* \\[4mm] \left(\dfrac{Ratio\,*}{\overline{Ratio}}\right)^{n} - 1 & if\ \overline{Ratio} > Ratio\,* \end{cases}$$

$$f_{CH4} = \frac{\alpha \cdot Q_{CH4}^{*}}{\overline{Q_{CH4}} + \alpha \cdot Q_{CH4}^{*}}$$

$$Indicator\ 1 = \begin{cases} f_{Ratio} \cdot f_{CH4} & if \quad f_{Ratio} > 0 \\ f_{Ratio} & if \quad f_{Ratio} \le 0 \end{cases}$$

où *Ratio\** et $Q^*_{CH4}$ sont les valeurs de référence respectivement du rapport d'alcalinité et du débit de méthane, et m, n et $\alpha$ sont des paramètres obtenus de façon empirique à partir d'ajustement de données expérimentales.

6. Procédé selon la revendication 5, **caractérisé en ce que** si la valeur de l'indicateur I est négative, le système est déstabilisé et procède à une restriction de la plage de la restriction la plus active, ce qui diminue la fonction d'objectif ; tandis que si la valeur de l'indicateur I est positive, le système est stable et les limites de la restriction la plus active sont augmentées en obtenant une valeur plus élevée de la fonction d'objectif.

7. Procédé selon la revendication 6, **caractérisé en ce que** l'action de contrôle modifie la limite de la restriction la plus active par une équation dans laquelle la nouvelle limite est calculée en tenant compte de la valeur actuelle de la limite de la restriction, de la plage de la restriction, de la différence entre les limites supérieure et inférieure de la restriction, et de l'indicateur I selon l'expression suivante :

$$\text{Limit}_{NEW} = Limit_{CURRENT} + indicator\ 1 \cdot \left( Limit_{UP} - Limit_{DOWN} \right)$$

où $\text{Limit}_{NEw}$ est la nouvelle limite, $\text{Limit}_{CURRENT}$ est la limite actuelle et $\text{Limit}_{UP}$ et $\text{Limit}_{DOWN}$ sont respectivement les limites supérieure et inférieure de la plage.

8. Procédé selon les revendications 6 à 7, **caractérisé en ce que** la modification des composants du mélange est effectuée tous les quarts du temps de séjour hydraulique (HRT).

9. Produit de programme d'ordinateur comprenant des instructions de programme pour amener un système informatique à exécuter le procédé de commande d'une installation de codigestion anaérobie selon l'une quelconque des revendications 1 à 8.

10. Moyen de stockage lisible par ordinateur sur lequel est stocké le produit de programme informatique de la revendication 9.

11. Onde porteuse de données portant le produit de programme informatique de la revendication 9.

101

102

Experimental data

Heuristic knoweldge

103

104

Objective Function

Linear restrictions

105

Linear Programming
Optimization

107

Optimal mixture and
HRT

106

Reduction of the most active restriction for a
subsequent optimization

Return to the last limits of conservative
restrictions on system stability

Diagnosis
Real Plant

Diagnosis
Virtual Plant

Stable
operation?

FIGURE 1

205

204

Indicator I

CONTROLLER

fCH4
fRatio

DIAGNOSIS

FLOW CH4

Alkalinity Ratio

Modification of
restrictions

202

BLENDER

xi (% vol.)

HRT

CH4 FLOW

Alkalinity Ratio

FILTERING

Linear programming
optimization method

201

203

ANAEROBIC DIGESTOR

FIGURE 2

16

**FIGURE 3**

FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **APPELS et al.** *Renewable and Sustainable Energy Reviews,* 2011, vol. 15, 4295-4301
- **KHALID et al.** *Waste management,* 2011, vol. 31, 1737-1744
- **MATA-ALVAREZ et al.** *Bioresource Technology,* 2000, vol. 74, 3-16
- **ALVAREZ et al.** *Bioresource Technology,* 2010, vol. 101, 1153-1158
- **BOE et al.** *Water Research,* 2010, vol. 44, 5973-5980
- **MADSEN et al.** *Renewable and Sustainable Energy Reviews,* 2011, vol. 15, 3141-3155
- **BATSTONE et al.** *Water Science and Technology,* 2002, vol. 45, 65-73
- **RODRIGUEZ et al.** *Water Science & Technology,* 2006, vol. 54, 57-62
- **GARCIA-GEN et al.** *Bioresource Technology,* 2013, vol. 147, 525-533